# EUROPEAN PATENT SPECIFICATION

(11) **EP 4 265 262 B1**
(45) Date of publication and mention of the grant of the patent: **11.02.2026**
(21) Application number: 21907162.8
(22) Date of filing: 17.12.2021
(51) Int. Cl.: A61K 33/06, A61K 38/14, A61K 9/00, A61P 31/04, A61P 1/12, A61K 9/16, A61K 9/19, A61K 33/08

(54) **PHARMACEUTICAL COMPOSITION FOR USE IN THE TREATMENT OF CDI COMPRISING CLAY MINERAL COMPLEX, AND METHOD FOR PRODUCING SAME**
PHARMAZEUTISCHE ZUSAMMENSETZUNG ZUR VERWENDUNG BEI DER BEHANDLUNG VON CDI MIT TONMINERALKOMPLEX, UND VERFAHREN ZUR HERSTELLUNG DAVON
COMPOSITION PHARMACEUTIQUE POUR UTILISATION DANS LE TRAITEMENT DE CDI COMPRENANT UN COMPLEXE MINÉRAL ARGILEUX, ET SON PROCÉDÉ DE PRODUCTION

(30) Priority: 18.12.2020 KR 20200178305
(43) Date of publication of application: 25.10.2023
(73) Proprietor: KOREA INSTITUTE OF GEOSCIENCE AND MINERAL RESOURCES, Yuseong-gu Daejeon 34132 (KR)
(72) Inventor: KANG, Il Mo, Seoul 07528 (KR); KWAK, Jin Hwan, Pohang-si Gyeongsangbuk-do 37590 (KR); KIM, Daniel, Pohang-si Gyeongsangbuk-do 37566 (KR); ROH, Ki Min, Daejeon 34061 (KR); KIM, Jae Hwan, Pohang-si Gyeongsangbuk-do 37571 (KR); SEO, Sung Man, Pohang-si Gyeongsangbuk-do 37883 (KR); KIM, Dae Young, Geumsan-gun Chungcheongbuk-do 32722 (KR)
(74) Representative: Berggren Oy
(86) International application number: PCT/KR2021/019293
(87) International publication number: WO 2022/131858

(56) References cited:
- KR-A- 20150 095 623
- KR-A- 20150 095 623
- KR-B1- 101 541 876
- US-A1- 2006 239 992
- US-A1- 2017 173 093
- US-A1- 2017 173 093
- WEESE J S ET AL: "Evaluation of in vitro properties of di-tri-octahedral smectite on clostridial toxins and growth", EQUINE VETERINARY JOURNAL, R & W PUBLICATIONS, SUFFOLK, GB, vol. 35, no. 7, 5 January 2010 (2010-01-05), pages 638 - 641, XP071652955, ISSN: 0425-1644, DOI: 10.2746/042516403775696384
- J. S. WEESE; N. M. COTE; R. V. G. DEGANNES: "Evaluation of in vitro properties of di‐tri‐octahedral smectite on clostridial toxins and growth", EQUINE VETERINARY JOURNAL., R & W PUBLICATIONS, SUFFOLK., GB, vol. 35, no. 7, 5 January 2010 (2010-01-05), GB , pages 638 - 641, XP071652955, ISSN: 0425-1644, DOI: 10.2746/042516403775696384

## Description

### Technical Field

The present disclosure relates to a pharmaceutical composition including clay minerals suitable for treating *Clostridioides difficile* infection (CDI) including a clay mineral complex, and a method for producing the same, and more particularly, to a pharmaceutical composition including a clay mineral complex including smectite capable of efficiently treating CDI in a smaller amount than the related art as a pharmaceutical composition for treating CDI and a method for producing the same.

### Background Art

*Clostridium difficile (C. difficile)* is an anaerobic gram-positive bacillus that forms spores and is widely distributed in nature. *C. difficile* is a disease that accompanies diarrhea in patients taking antibiotics for therapeutic purposes and is known as the most common cause of hospital-acquired diarrheal disease. A pathogenic *C*. *difficile* strain produces various toxins such as enterotoxin and cytotoxin, and may cause diarrhea and inflammation, and the diarrhea may be various from fluid loss for several days to life-threatening pseudomembranous colitis.

Reports of antibiotics-resistant *C*. *difficile* have recently been increased, and the US CDC has defined *C*. *difficile* as an urgent threat cause to healthcare-associated infections (ANTIBIOTIC RESISTANCE THREATS IN THE UNITED STATES 2019).

*Clostridioides difficile* infection (CDI) is one of the most common causes of hospital-associated infections, and occurs when the composition of the normal intestinal flora of patients receiving antibiotics is changed and *C*. *difficile* colonizes, and causes diseases such as bacterial colitis.

Meanwhile, smectite is a leaflike silicate mineral constituting one unit layer (2 : 1 layer) by combining a tetrahedral sheet consisting of Si, Al, and Fe with two octahedral sheet consisting of Al, Mg, and Fe up and down in a sandwich shape. The smectite unit layer has a negative charge, which is generated when tetrahedral Si having a tetravalent positive charge is isomorphic-substituted with Al or Fe having a trivalent positive charge or octahedral Al or Fe³⁺ having a trivalent positive charge is isomorphic-substituted with Mg or Fe²⁺ having a bivalent positive charge. Cations are induced between unit layers through a negative charge generated from the unit layer, which means that the smectite may be used as a drug carrier. Therefore, the use of smectite as a drug delivery vehicle has recently attracted great attention, and many studies have been reported on smectite hybrids into which drugs are inserted for controlled delivery and release of donepezil, lincomycin, chlorhexidine acetate, and tetracycline.

Meanwhile, bentonite, a natural clay mineral included in smectite-based minerals, plays an important role in tissue engineering due to excellent characteristics such as high cation exchange and high surface area. A microstructure thereof is formed of a stacked flat plate structure. Each flat plate consists of three sandwiched layers having a central octahedral alumina (Al₂O₃) layer, and two tetrahedral silica (SiO₂) layers. In addition, bentonite has a high swelling ratio, a high adsorption capacity and a drug delivery capacity. The characteristics of bentonite include a large interlayer space, a cation exchange capacity, an antibacterial activity, drug-delivery and release, swelling and hygroscopicity, gel formation, and a capacity serving as cell-centric adsorption sites enhancing cell adhesion and cell proliferation.

In addition, compared to carbon-based materials and gold nanoparticles, bentonite is a natural clay that may be decomposed into chemical elements such as magnesium and calcium, and thus is harmless to the human body and no toxicity has been reported, making it suitable for tissue regeneration research.

Meanwhile, it is already known that vancomycin, used as a type of antibiotic, has an excellent antibacterial effect against *C*. *difficile,* but has no inhibitory effect against the toxin of *C*. *difficile,* which plays an important role in the pathological action of bacterial colitis. On the other hand, smectite, which is used as a therapeutic agent for colitis accompanied by diarrhea and uses bentonite as a main component, is effective in removing the toxin of *C*. *difficile.*

US 2017/173093 A1 and KR 2015 0095623 A disclose smectite and bentonite compositions for use in the treatment of *C*. *difficile* infections.

Accordingly, the present inventors have studied a composition suitable for the treatment of *C*. *difficile,* produced a complex of smectite-based clay minerals comprising bentonite with an excellent toxin removal effect and antibiotics with an excellent antibacterial effect against *C*. *difficile,* confirmed that the complex exhibited an excellent therapeutic effect with a pharmacological synergy action against CDI, and then completed the present disclosure.

### Disclosure of the Invention

### Technical Goals

An aspect of the present disclosure is to provide a pharmaceutical composition for treating CDI including a clay mineral complex and a method for producing the same, capable of significantly reducing the used amount of antibiotics previously used for CDI as well as shortening a treatment period by a synergistic action of the drug complex and also greatly reducing side effects caused by the excessive used amount of antibiotics.

However, the technical goals to be achieved are not limited to the above-mentioned aspects, and other goals not mentioned may be clearly understood by one of ordinary skill in the art from the following description.

### Technical Solutions

According to claim 1, there is provided a pharmaceutical composition for use in treating *Clostridioides difficile* infection (CDI) including a clay mineral complex including smectite.

According to another example of the present disclosure, there is provided a composition for oral administration for treating CDI including a clay mineral complex including smectite.

According to claim 8 there is provided a method for producing a pharmaceutical composition for treating *Clostridioides difficile* infection (CDI) including:
1) preparing a bentonite suspension and a vancomycin solution using distilled water;
2) mixing and stirring the prepared bentonite suspension and vancomycin solution;
3) centrifuging and washing the stirred solution; and
4) freeze-drying the product.

### Effects

The present disclosure relates to a composition for treating CDI including a clay mineral complex, and a method for producing the same, as defined in claims 1 and 8, respectively. The composition includes a complex of antibiotics comprising vancomycin with an excellent antibacterial effect against

### C. difficile and clay minerals comprising bentonite with an excellent toxin removal effect.

Accordingly, the composition has an excellent treatment effect on CDI even with a significantly smaller amount of each of the existing antibiotics and clay minerals than the maximum recommended daily intake thereof, and has the effect of reducing side effects according to the massive intake.

### Brief Description of Drawings

FIG. 1 is a graph showing results obtained by performing X-ray diffraction on a complex prepared by mixing Ca-type bentonite and vancomycin for each concentration.
FIG. 2 is a graph showing results obtained by performing X-ray diffraction on a complex prepared by mixing Na-type bentonite and vancomycin for each concentration.

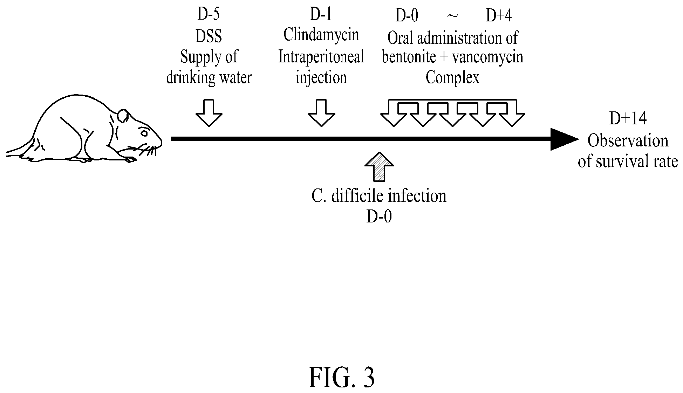

FIG. 3 is a diagram sequentially illustrating a series of experiments of being treated with dextran sodium sulfate (DSS), being infected with *C*. *difficile,* being administered with a bentonite-vancomycin complex, and observing survival rates, in order to produce a mouse acute CDI model.
FIG. 4 is a graph showing survival rates of mice at D - 0 and D + 14 according to bentonite contents of 0, 125, 250, 500, 1000, and 2000 mg/kg.
FIG. 5 is a graph showing survival rates of mice at D - 0 and D + 14 according to vancomycin contents of 0, 2, 20, and 200 mg/kg.
FIG. 6 is a graph showing survival rates at D - 0 and D + 14 according to mixing ratios and types of vancomycin and bentonite.

### Best Mode for Carrying Out the Invention

Hereinafter, the present disclosure will be described in detail.

According to claim 1, there is provided a pharmaceutical composition for treating *Clostridioides difficile* infection (CDI) including a clay mineral complex containing smectite.

In general, the clay minerals have a layered structure, that is, a plate-like structure in which crystal units formed by combining silica sheets and alumina sheets are stacked, and in the clay minerals having interlayer expansibility among these clay minerals, since the binding force between the crystal units is weak due to no hydrogen bond between the crystal units, moisture is introduced between the crystal units to be expanded. Therefore, it is possible to easily introduce even ions having relatively large sizes between the crystal units of the clay minerals having interlayer expansibility. Meanwhile, in the clay minerals having interlayer expansibility, tetrahedral Si having a tetravalent positive charge is isomorphic-substituted with Al or Fe having a trivalent positive charge or octahedral Al or Fe³⁺ having a trivalent positive charge is isomorphic-substituted with Mg or Fe²⁺ having a bivalent positive charge to generate a negative layer charge, but cations such as calcium ions (Ca²⁺), magnesium ions (Mg⁺), sodium ion (Na⁺), potassium ions (K⁺), and the like are bound to each other between the layers or on the surface to have entirely electric neutrality.

In the present disclosure, the clay minerals comprise smectite-based minerals, including bentonite as a main component. By having these specific clay minerals and shapes according thereto, bacteria such as C. *difficile* that cause bacterial colitis may be adsorbed to the clay minerals to be removed, and thus, there is an advantage of being able to be easily used for CDI treatment.

In particular, using the bentonite as a clay mineral, the bentonite may be at least one selected from the group consisting of Na-type bentonite, Ca-type bentonite, and Mg-type bentonite, and the Na-type bentonite may be more suitable in terms of suitability for holding a drug, and the Ca-type bentonite may be more suitable in terms of an antiinflammatory effect.

Meanwhile, the clay mineral complex further includes antibiotics suitable for treating CDI, at least comprising vancomycin, and may further be desirably at least one selected from the group consisting of metronidazol, fidaxomicin and teicoplanin.

The vancomycin is a substance that has its limited use (maximum daily intake of 33 mg/kg or less) by requiring careful use due to the recent emergence of resistant *Enterococci* and *Staphylococci,* and may also accompany side effects due to overuse. These side effects may include anaphylaxis, toxic epidermal necrosis, erythema multiforme, erythema syndrome, hyperplasia, thrombocytopenia, neutropenia, leukopenia, tinnitus, dizziness, and ototoxicity, and cause severe thrombocytopenia and hemorrhage by inducing a platelet-reactive antibody in a patient and may also cause petechiae, ecchymosis, and purpura.

Therefore, as vancomycin, which may have these side effects, is used as the antibiotics of the complex according to the present disclosure, compared to an amount administered as a single component of vancomycin, there is an advantage that the complex may be prepared and administered for treatment in a relatively small amount, and may exhibit a superior CDI therapeutic effect as compared to a case of administering a single component.

In the pharmaceutical composition for treating the CDI provided according to the present disclosure, a ratio of the content concentration of bentonite and the content concentration of vancomycin included in the pharmaceutical composition is 9 : 1 to 11 : 1, preferably 10 : 1, and particularly, for example, the content concentration of bentonite may be 50 mg/kg and the content concentration of antibiotics may be 5 mg/kg.

Meanwhile, according to another aspect of the present disclosure, in the composition for oral administration for treating CDI, the composition includes a clay mineral complex containing smectite.

According to yet another aspect of the present disclosure, there is provided a method for producing a pharmaceutical composition for treating *Clostridioides difficile* infection (CDI) including:
1) preparing a bentonite suspension and a vancomycin solution using distilled water;
2) mixing and stirring the prepared bentonite suspension and vancomycin solution;
3) centrifuging and washing the stirred solution; and
4) freeze-drying the product,
wherein the bentonite suspension and the vancomycin solution in 1) have the same concentration, and wherein the bentonite suspension and the vancomycin solution in 2) are mixed at 10 a volume ratio of 9 : 1 to 11 : 1.

In particular, as described above, the concentrations of the bentonite suspension and the vancomycin solution are the same, the bentonite suspension and the vancomycin solution are mixed at a volume ratio of 9 : 1 to 11 : 1, so that the ratio of the content concentration of bentonite and the content concentration of antibiotics in the finally formed pharmaceutical composition is 9 : 1 to 11 : 1, desirably 10 : 1.

Meanwhile, in the method for producing the pharmaceutical composition for treating CDI, before step 4), step 3) may be repeated two or more times, desirably 3 times or more, more desirably 3 times or more and 5 times or less.

Hereinafter, Examples will be described in detail with reference to the accompanying drawings. However, since various modifications may be made to Examples, the scope of the present disclosure is not limited or restricted by these Examples.

The singular expression includes the plural expression unless the context clearly dictates otherwise. In the present disclosure, it should be understood that term "including" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which Examples pertain. Terms which are defined in a generally used dictionary should be interpreted to have the same meaning as the meaning in the context of the related art, and are not interpreted as ideal or excessively formal meanings unless otherwise defined in the present specification.

In addition, in the description with reference to the accompanying drawings, like components designate like reference numerals regardless of reference numerals and a duplicated description thereof will be omitted.

In describing the components of the Examples of the present disclosure, terms including first, second, A, B, (a), (b), and the like may be used.

Components included in any one Example and components having a common function will be described using the same names in other Examples. Unless otherwise stated, descriptions described in any one Example may also be applied to other Examples, and detailed descriptions in the overlapping range will be omitted.

Next, a pharmaceutical composition including a clay mineral complex according to an Example of the present disclosure and a method for producing the same will be described with reference to the drawings.

### - Examples

### 1) Experimental Example 1: Preparation of bentonite-vancomycin complex

First, a solution having a concentration of 100 mg/g of vancomycin was prepared using distilled water. Thereafter, in the same manner, a suspension having a concentration of 100 mg/g of Na-type bentonite was prepared, and the vancomycin solution and the Na-type bentonite suspension were stirred for 1 hour at a volume ratio of 1 : 10. The stirred solution was centrifuged at 3000 rpm and washed. After repeating the process a total of 3 times, lyophilization was performed to prepare a bentonite-vancomycin complex.

### 2) Experimental Example 2: X-ray diffraction analysis of complex

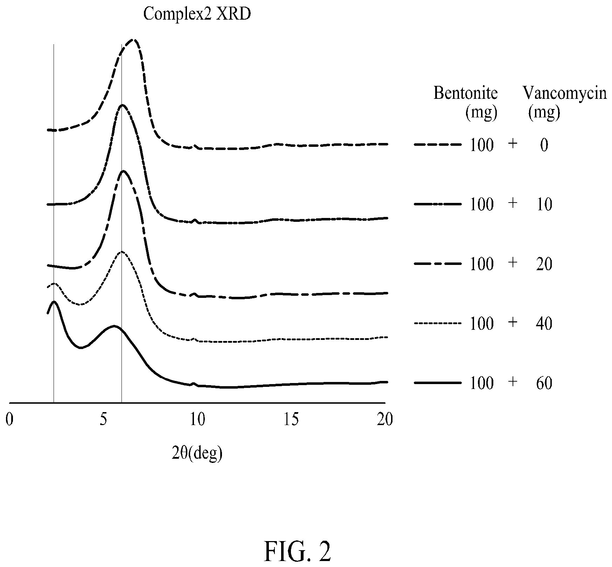

According to the preparation method of Experimental Example 1, X-ray diffraction analysis was performed for the Na-type bentonite-vancomycin complex obtained for each content of bentonite + vancomycin (100 mg + 0 mg, 100 mg + 10 mg, 100 mg + 20 mg, 100 mg + 40 mg, and 100 mg + 60 mg), and the results were illustrated in FIG. 2.

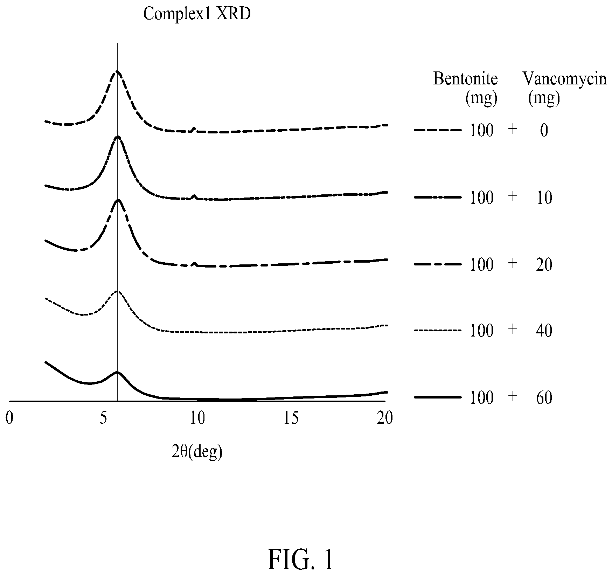

In addition, in the same manner as the preparation method of Experimental Example 1, X-ray diffraction analysis was performed for the Ca-type bentonite-vancomycin complex obtained for each content of bentonite + vancomycin (100 mg + 0 mg, 100 mg + 10 mg, 100 mg + 20 mg, 100 mg + 40 mg, and 100 mg + 60 mg), and the results were illustrated in FIG. 1.

FIG. 1 illustrates that the atomic spacing (d-spacing) did not change in all experimental groups in an X-ray diffraction analysis technique, and as a result, it can be seen that the drug was not intercalated in bentonite.

FIG. 2 illustrates that the atomic spacing (d-spacing) for each experimental group was changed, and as a result, it can be seen that the drug was intercalated in bentonite.

### 3) Experimental Example 3: Experiment for confirming mouse survival rate according to bentonite content

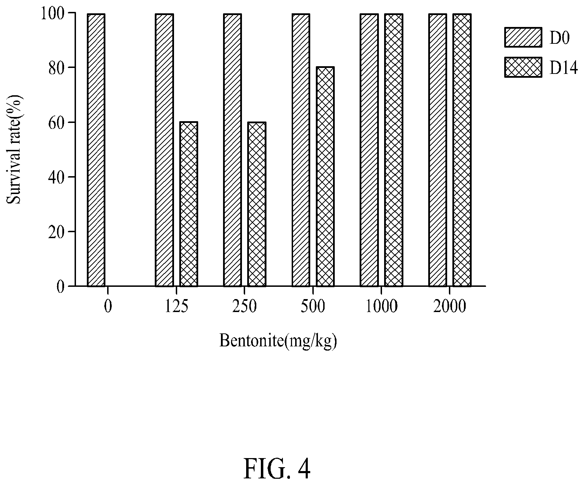

To set a mixing ratio of a bentonite-antibiotics complex, in a mouse acute CDI model using dextran sulfate sodium (DSS), bentonite was orally administered at concentrations of 0, 125, 250, 500, 1000, and 2000 mg/kg, respectively, and the results were shown in Table 1 below and FIG. 4.

**[Table 1]**

| Bentonite (mg/kg) | Mouse survival rate (%) according to bentonite dose | | | | | |
|---|---|---|---|---|---|---|
| | 0 | 125 | 250 | 500 | 1000 | 2000 |
| D-0 (D-day) | 5/5 (individual number) (100%) | 5/5 (100%) | 5/5 (100%) | 5/5 (100%) | 5/5 (100%) | 5/5 (100%) |
| D+14 | 0/5 (0%) | 3/5 (60%) | 3/5 (60%) | 4/5 (80%) | 5/5 (100%) | 5/5 (100%) |

As illustrated in Table 1 and FIG. 4, as a result of confirming a treatment effect of bentonite alone by concentration, in the case of 1000 mg/kg and 2000 mg/kg, the CDI treatment effect of 100% survival rate was confirmed, and the treatment effect (60, 60, and 80%, respectively) of 50% or more was confirmed even at 125, 250, and 500 mg/kg.

That is, when the dose concentration of bentonite was 0, 125, 250, 500, 1000, and 2000 mg/kg, it was confirmed that the survival rates thereof were 0, 60, 60, 80, 100, and 100%, respectively.

### 4) Experimental Example 4: Experiment for confirming mouse survival rate according to vancomycin content

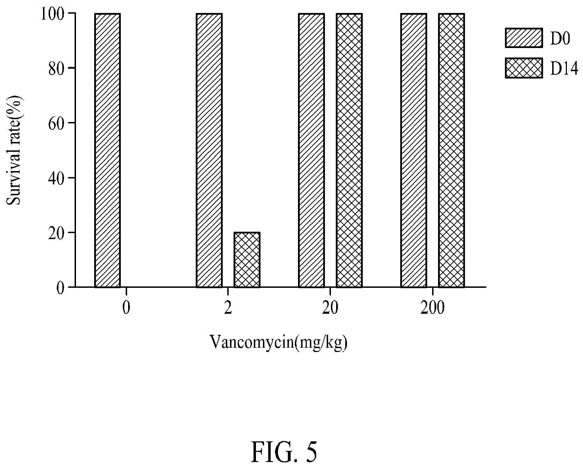

In order to set the mixing ratio of the bentonite-antibiotics complex, vancomycin was orally administered at concentrations of 2, 20, and 200 mg/kg in a mouse acute CDI model using DSS, and the results were shown in Table 2 below and FIG. 5.

**[Table 2]**

| Vancomycin (mg/kg) | Mouse survival rate (%) according to vancomycin dose | | | |
|---|---|---|---|---|
| | 0 | 2 | 20 | 200 |
| D-0 | 5/5 (100%) | 5/5 (100%) | 5/5 (100%) | 5/5 (100%) |
| D+14 | 0/5 (0%) | 1/5 (20%) | 5/5 (100%) | 5/5 (100%) |

As shown in Table 2 above and FIG. 5, as a result of confirming the treatment effect of vancomycin alone by concentration, it was confirmed that the treatment effect of survival rate 100% was exhibited at the concentrations of 20 mg/kg and 200 mg/kg, and the treatment effect of survival rate 20% was exhibited at 2 mg/kg.

That is, when the dose concentration of vancomycin was 0, 2, 20, and 200 mg/kg, it was confirmed that the survival rates thereof were 0, 20, 100, and 100%, respectively.

### 5) Experimental Example 5: Experiment for confirming drug synergy effect according to mixture of bentonite-antibiotics complex

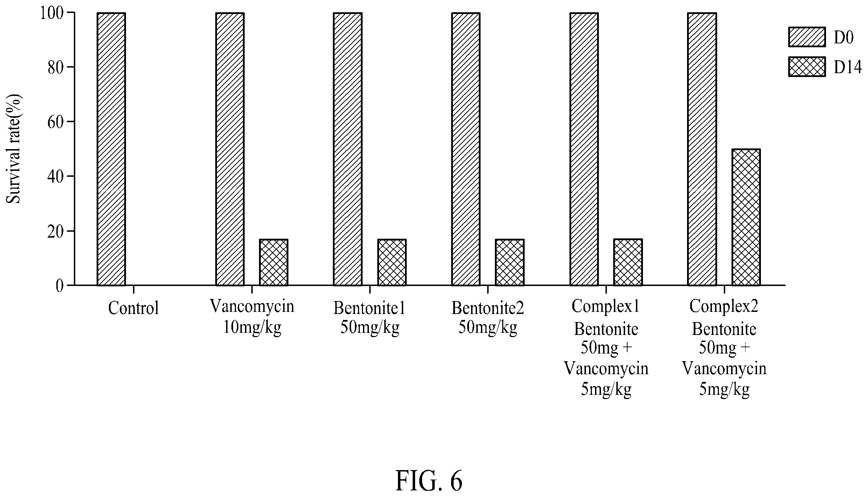

In order to confirm a synergistic effect on CDI treatment through the bentonite-antibiotics complex, according to the daily intake, the bentonite was administered at a dose concentration of 50 mg/kg and the vancomycin was administered at a dose concentration of 5 mg/kg, and the survival rates of mice were confirmed as compared to cases of administering 10 mg/kg of vancomycin alone, 50 mg/kg of bentonite alone, and saline alone as control groups thereto. The results were shown in Table 3 below and FIG. 6.

The abbreviations shown in Table 3 and FIG. 6 were as follows:
V10: Vancomycin-administered group at 10 mg/kg concentration per mouse unit weight
BT: Ca-type bentonite-administered group
NBT: Na-type bentonite-administered group
BTV: Administered group of vancomycin and Ca-type bentonite complex
NBTV: Administered group of vancomycin and Na-type bentonite complex

**[Table 3]**

| Experimental drug | Mouse survival rate (%) according to type of administered drug | | | | | |
|---|---|---|---|---|---|---|
| | Saline | V10 | BT | NBT | BTV | NBTV |
| D-0 | 6/6 (100%) | 6/6 (100%) | 6/6 (100%) | 6/6 (100%) | 6/6 (100%) | 6/6 (100%) |
| D+14 | 0/6 (0%) | 1/6 (17%) | 1/6 (17%) | 1/6 (17%) | 1/6 (17%) | 3/6 (50%) |

As illustrated in Table 3 above and FIG. 6, it was confirmed that in the case of administering a complex prepared by 50 mg/kg of Na-type bentonite and 5 mg/kg of vancomycin (NBTV), a higher survival rate was shown than a case of administering 50 mg/kg of bentonite alone (BT and NBT) or a case of administering 10 mg/kg of vancomycin alone (V10).

Accordingly, it could be confirmed that the complex of bentonite (clay mineral) and vancomycin (antibiotic) generated a synergistic effect compared to the case of administering each substance alone to be more effective on CDI treatment.

## Claims

1. A pharmaceutical composition for use in treating *Clostridioides difficile* infection (CDI) comprising:
a clay mineral complex comprising smectite, wherein the smectite comprises bentonite, and antibiotics, wherein the antibiotics comprise at least vancomycin,
wherein a weight ratio of a content concentration of the bentonite and a content concentration of the vancomycin is 9 : 1 to 11 : 1.

2. The pharmaceutical composition for use of claim 1, wherein the bentonite comprises at least one selected from the group consisting of Na-type bentonite, Ca-type bentonite, and Mg-type bentonite.

3. The pharmaceutical composition for use of claim 1 or 2, wherein the bentonite is Na-type bentonite.

4. The pharmaceutical composition for use of claim 1, wherein the antibiotics further comprise at least one selected from the group consisting of metronidazol, fidaxomicin and teicoplanin.

5. The pharmaceutical composition for use of claim 1, wherein a weight ratio of a content concentration of the bentonite and a content concentration of the antibiotics is 9 : 1 to 11 : 1.

6. The pharmaceutical composition for use of claim 1, wherein a content concentration of the bentonite is 50 mg/kg and a content concentration of the vancomycin is 5 mg/kg.

7. The pharmaceutical composition for use of any one of claims 1 to 6, wherein the pharmaceutical composition is administered orally.

8. A method for producing a pharmaceutical composition for use in treating *Clostridioides difficile* infection (CDI) comprising:
1) preparing a bentonite suspension and a vancomycin solution using distilled water;
2) mixing and stirring the prepared bentonite suspension and vancomycin solution;
3) centrifuging and washing the stirred solution; and
4) freeze-drying the product,
wherein the bentonite suspension and the vancomycin solution in 1) have the same concentration, and
wherein the bentonite suspension and the vancomycin solution in 2) are mixed at a volume ratio of 9 : 1 to 11 : 1.

9. The method of claim 8, wherein before 4), 3) is repeated 2 times or more and 5 times or less.

## Patentansprüche

1. Pharmazeutische Zusammensetzung zur Verwendung bei der Behandlung einer *Clostridioides difficile-Infektion* (CDI), umfassend:
einen Tonmineralkomplex, der Smectit umfasst, wobei das Smectit Bentonit umfasst, und Antibiotika, wobei die Antibiotika mindestens Vancomycin umfassen,
wobei ein Gewichtsverhältnis einer Gehaltskonzentration des Bentonits und einer Gehaltskonzentration des Vancomycins 9:1 bis 11:1 beträgt.

2. Pharmazeutische Zusammensetzung zur Verwendung nach Anspruch 1, wobei der Bentonit mindestens einen, ausgewählt aus der Gruppe, bestehend aus Na-Typ-Bentonit, Ca-Typ-Bentonit und Mg-Typ-Bentonit, umfasst.

3. Pharmazeutische Zusammensetzung zur Verwendung nach Anspruch 1 oder 2, wobei der Bentonit ein Na-Typ-Bentonit ist.

4. Pharmazeutische Zusammensetzung zur Verwendung nach Anspruch 1, wobei die Antibiotika weiter mindestens eines, ausgewählt aus der Gruppe, bestehend aus Metronidazol, Fidaxomicin und Teicoplanin, umfassen.

5. Pharmazeutische Zusammensetzung zur Verwendung nach Anspruch 1, wobei ein Gewichtsverhältnis einer Gehaltskonzentration des Bentonits und einer Gehaltskonzentration der Antibiotika 9 : 1 bis 11 : 1 beträgt.

6. Pharmazeutische Zusammensetzung zur Verwendung nach Anspruch 1, wobei eine Gehaltskonzentration des Bentonits 50 mg/kg und eine Gehaltskonzentration des Vancomycins 5 mg/kg beträgt.

7. Pharmazeutische Zusammensetzung zur Verwendung nach einem der Ansprüche 1 bis 6, wobei die pharmazeutische Zusammensetzung oral verabreicht wird.

8. Verfahren zur Herstellung einer pharmazeutischen Zusammensetzung zur Verwendung bei der Behandlung von *Clostridioides difficile-Infektionen* umfassend:
1) das Herstellen einer Bentonitsuspension und einer Vancomycinlösung unter Verwendung von destilliertem Wasser;
2) das Mischen und Rühren der hergestellten Bentonitsuspension und Vancomycinlösung;
3) das Zentrifugieren und Waschen der gerührten Lösung; und
4) das Gefriertrocknen des Produkts,
wobei die Bentonitsuspension und die Vancomycinlösung in 1) die gleiche Konzentration aufweisen und
wobei die Bentonitsuspension und die Vancomycinlösung in 2) in einem Volumenverhältnis von 9:1 bis 11:1 gemischt werden.

9. Verfahren nach Anspruch 8, wobei vor 4) 3) 2 Mal oder mehr und 5 Mal oder weniger wiederholt wird.

## Revendications

1. Composition pharmaceutique destinée à être utilisée pour le traitement de l'infection *à Clostridioides difficile* (ICD) comprenant :
un complexe minéral argileux comprenant de la smectite, dans laquelle la smectite comprend de la bentonite, et des antibiotiques, dans laquelle les antibiotiques comprennent au moins de la vancomycine,
dans laquelle le rapport pondéral de la concentration en bentonite et de la concentration en vancomycine est de 9:1 à 11:1.

2. Composition pharmaceutique destinée à être utilisée selon la revendication 1, dans laquelle la bentonite comprend au moins un élément choisi dans le groupe constitué de la bentonite de type Na, de la bentonite de type Ca et de la bentonite de type Mg.

3. Composition pharmaceutique destinée à être utilisée selon la revendication 1 ou 2, dans laquelle la bentonite est une bentonite de type Na.

4. Composition pharmaceutique destinée à être utilisée selon la revendication 1, dans laquelle les antibiotiques comprennent en outre au moins un élément choisi dans le groupe constitué du métronidazole, de la fidaxomicine et de la teicoplanine.

5. Composition pharmaceutique destinée à être utilisée selon la revendication 1, dans laquelle le rapport pondéral de la concentration en bentonite et de la concentration en antibiotiques est de 9:1 à 11:1.

6. Composition pharmaceutique destinée à être utilisée selon la revendication 1, dans laquelle la concentration en bentonite est de 50 mg/kg et la concentration en vancomycine est de 5 mg/kg.

7. Composition pharmaceutique destinée à être utilisée selon l'une quelconque des revendications 1 à 6, dans laquelle la composition pharmaceutique est administrée par voie orale.

8. Procédé de fabrication d'une composition pharmaceutique destinée à être utilisée pour le traitement de l'infection à *Clostridioides difficile* (ICD) comprenant :
1) la préparation d'une suspension de bentonite et d'une solution de vancomycine à l'aide d'eau distillée ;
2) le mélange et l'agitation de la suspension de bentonite préparée et de la solution de vancomycine ;
3) la centrifugation et le lavage de la solution agitée ; et
4) la lyophilisation du produit,
dans lequel la suspension de bentonite et la solution de vancomycine en 1) présentent la même concentration, et
dans lequel la suspension de bentonite et la solution de vancomycine dans 2) sont mélangées dans un rapport volumique de 9:1 à 11:1.

9. Procédé selon la revendication 8, dans lequel avant 4), 3) est répété 2 fois ou plus et 5 fois ou moins.
